# EUROPÄISCHE PATENTANMELDUNG

(11) **EP 3 366 371 A1**
(43) Veröffentlichungstag der Anmeldung: **29.08.2018**
(21) Anmeldenummer: 17158520.1
(22) Anmeldetag: 28.02.2017
(51) Int. Cl.: B01J 20/26, C07C 303/04, C07C 303/32, C07C 309/17, C07C 303/42

(54) **POLYMERABSORBER UND HERSTELLUNG EINES POLYMERABASORBERS**

(71) Anmelder: ESIM Chemicals GmbH, 4020 Linz (AT)
(72) Erfinder: STÖGLEHNER, Alex, 4210 Gallneukirchen (AT); KASSLER, Alexander, 1150 Wien (AT); KÖNIG, Michael, 4020 Linz (AT); KOGLER, Martina, 4030 Linz (AT); HINTERWIRTH, Helmut, 4020 Linz (AT); SCHUECKER, Raffael, 4020 Linz (AT); HÄUBL, Martin, 4040 Linz (AT); DOMKE, Lutz, 4020 Linz (AT); HOLUB, Bernhard, 4072 Strassham (AT)
(74) Vertreter: Prüfer & Partner mbB Patentanwälte · Rechtsanwälte

(57) **Zusammenfassung**

Ein Absorber, der durch eine Vernetzung, insbesondere durch eine thermische Vernetzung, eines Polymermaterials mit einem Sulfobernsteinsäurematerial erhältlich ist. Das Sulfobernsteinsäurematerial ist frei oder im Wesentlichen frei von Alkalimetallionen.

## Beschreibung

Vorliegende Erfindung bezieht sich auf einen Absorber, insbesondere einen Superabsorber. Ferner bezieht sich die Erfindung auf die Verwendung eines Absorbers und auf die Verwendung eines Sulfobernsteinsäurematerials zur Herstellung eines Absorbers sowie auf Verfahren zum Herstellen eines Absorbers.

Unter Absorbern werden Polymermaterialien mit einer Kapazität zur Anlagerung bzw. Absorption von Flüssigkeiten, insbesondere polaren Flüssigkeiten und vor allem wässrige Flüssigkeiten, verstanden. Absorber sind somit in der Lage, Flüssigkeiten aufzunehmen und zurückzuhalten. Bei einer polaren Flüssigkeit handelt es sich insbesondere um Wasser und wässrige Flüssigkeiten, beispielsweise wässrige Lösungen oder Dispersionen organischer und/oder anorganischer Substanzen. Im Falle von Superabsorbern ist die Menge der Flüssigkeit, die aufgenommenen und zurückgehaltenen werden kann, groß, in der Regel ein Vielfaches des Eigengewichts des absorbierenden Kunststoffmaterials.

Bei Absorbern handelt es sich insbesondere um ein poröses und/oder faserartiges und quervernetztes Polymer. Der Begriff Polymer umfasst dabei Homopolymere, alle Arten von Co-Polymeren und Polyblends.

Absorber und Superabsorber sind grundsätzlich bekannt. Beispielhaft wird auf F.L. Buchholz und AT. Graham, "Modern Superabsorbent Polymer Technology", Wiley-VCH, 1998, Seiten 69 bis 117 verwiesen.

Absorber und insbesondere Superabsorber werden beispielsweise in Babywindeln, Damenbinden, Inkontinenzprodukten und Verbandmaterial zum Aufnehmen von Körperausscheidungen, beispielsweise Harn, und anderen Körperflüssigkeiten, beispielsweise Blut, verwendet. Ferner werden Absorber und insbesondere Superabsorber beispielsweise zur Herstellung gelbildender Löschmittel zur Brandbekämpfung, zur Herstellung von Gelbetten (spezielle Art von Wasserbetten) und zur Erhöhung der Wasserspeicherkapazität von Böden zum Kultivieren von Nutz- und Zierpflanzen verwendet.

Aus der WO 2007/098932 A2 ist ein Verfahren zur Herstellung einer superabsorbierenden Zusammensetzung bekannt, nach welchem ein Hydrogel durch radikalische Polymerisation einer wässrigen, mindestens ein Monomer beinhaltenden Monomerlösung hergestellt wird, dieses Hydrogel unter Erhalt eines wasserabsorbierenden Polymergebildes getrocknet wird und dieses wasserabsorbierende Polymergebilde oberflächenvernetzt wird. Ferner werden in der WO 2007/098932 A2 verschiedene Beispiele genannt, in denen eine Stärkeverbindung eingearbeitet wird. Mit einer derartigen superabsorbierenden Zusammensetzung sollen in ihrer Umweltverträglichkeit verbesserte Hygieneartikel zugänglich sein.

Aus der WO 2011/141522 A1 ist ein superabsorbierendes Material umfassend ein bioabbaubares vernetztes Polymer bekannt, wobei als geeignete Vernetzer genannt werden: Verbindungen mit mindestens zwei polymerisierbaren Doppelbindungen und Verbindungen mit mindestens einer polymerisierbaren Doppelbindung und mindestens einer weiteren funktionellen Gruppe, die gegenüber Säuregruppen reaktiv ist, Mono-, Di- und Polyester der Acrylsäure, Methacrylsäure, Itaconsäure und Maleinsäure, Mono-, Di- und Polyester von den mehrwertigen Alkoholen Butandiol, Hexandiol, Polyethylenglykol, Trimethylolpropan, Pentaerythrit, Glycerin und Polyglycerin, sowie die daraus resultierenden oxalkylierten Homologen, die Ester dieser Säuren mit Allylalkohol und seinen oxalkylierten Homologen, N-Diallylacrylamid, Diallylphthalat, Triallylcitrat, Trimonoallyl-polyethylenglykolethercitrat, Allylacrylamid, Allylether von Di- und Polyolen und deren Oxethylate, Diamine und deren Salze mit mindestens zwei ethylenisch ungesättigten Substituenten, Di- und Triallylamin, Tetraallylammoniumchlorid, 1,4-Butandioldiacrylat (BDDA), Methylenbisacrylamid und Allylmethacrylat (ALMA). Dadurch soll ein superabsorbierendes Material zugänglich sein, das nach Erfüllung seiner Aufgabe biologisch abbaubar ist.

Die im Stand der Technik bekannten Absorber und Superabsorber weisen häufig kein definiertes Quellverhalten auf, d.h. es ist beispielsweise nicht möglich, das absorbierende Grundmaterial - insbesondere bedingt durch dessen Herstellung - so zu präparieren, dass eine definierte oder gewünschte Menge der zu absorbierenden Flüssigkeit von einer vorgegebenen Menge des Absorbers oder Superabsorbers aufgenommen wird. Folglich kann die Saugfähigkeit von Produkten, beispielsweise Hygieneartikeln, die mit herkömmlichen Absorbern oder Superabsorbern hergestellt sind, nicht oder nicht genau reproduzierbar eingestellt werden. Um sicherzustellen, dass derartige Produkte trotz der schlechten Reproduzierbarkeit der Saugfähigkeit ein gewünschtes Mindestmaß an Saugfähigkeit aufweisen, wird herkömmlich häufig ein großer Überschuss an Absorbern oder Superabsorbern eingesetzt, was aus ökologischen und ökonomischen Gründen nachteilig ist. Ferner ist es herkömmlich nur unzureichend oder nicht zufriedenstellend möglich, zu bestimmen, wie sich das Volumen eines Absorbers oder Superabsorbers bei der Flüssigkeitsaufnahme verändert und mit welcher Geschwindigkeit die Flüssigkeitsaufnahme erfolgt. Folglich ist im Falle von unter Verwendung herkömmlicher Absorber und Superabsorber hergestellten Produkten nicht nur die Saugfähigkeit schlecht reproduzierbar, sondern es ist bei solchen Produkten auch nicht in reproduzierbarer Weise vorherzubestimmen, ob die Aufnahme von Flüssigkeit schnell oder langsam erfolgt und wie stark dabei die Volumenzunahme ist.

Die im Stand der Technik bekannten Absorber und Superabsorber sind außerdem nur zum Teil biologisch abbaubar. Ein rascher und nach Möglichkeit vollständiger biologischer Abbau von Absorbern und Superabsorbern ist jedoch im Hinblick auf den Umweltschutz und die Verbraucherakzeptanz vorteilhaft.

Unter der "biologischen Abbaubarkeit" eines Stoffes wird das Vermögen dieses Stoffes, unter Umweltbedingungen zu einfacher aufgebauten Strukturen, bevorzugt zu natürlich vorkommenden Endprodukten, abgebaut zu werden, verstanden. Daran können neben biologischen Prozessen beispielsweise auch chemische Prozesse (beispielsweise Hydrolysereaktionen) und/oder physikochemische Prozesse (beispielsweise Auflösung) und/oder photochemische Prozesse (beispielsweise Photodegradation) beteiligt sein. Unter "Umweltbedingungen" werden dabei insbesondere auch die in einer Kompostieranlage und/oder einem Faulbehälter vorherrschenden Bedingungen verstanden.

Eine Aufgabe der vorliegenden Erfindung besteht in der Bereitstellung eines Absorbers und insbesondere eines Superabsorbers, der einzelne und insbesondere alle der vorgenannten Nachteile des Stands der Technik nicht aufweist, und eines Verfahrens zu dessen Herstellung. Der bereitzustellende Absorber oder Superabsorber soll insbesondere gut definierbar und einstellbar herzustellen sein und ein definiertes Quellverhalten aufweisen. Ferner ist es erwünscht, gleichzeitig eine biologische Abbaubarkeit zu ermöglichen.

Die Aufgabe wird gelöst durch einen Absorber gemäß Anspruch 1, eine Verwendung eines Absorbers gemäß Anspruch 9, eine Verwendung eines Sulfobernsteinsäurematerials gemäß Anspruch 10, ein Verfahren gemäß Anspruch 11, ein Verfahren gemäß Anspruch 13 und ein Verfahren gemäß Anspruch 14.

Die in den jeweiligen Unteransprüchen festgelegten Merkmale und die in der Beschreibung angeführten Merkmale stellen Weiterbildungen des in den unabhängigen Ansprüchen definierten Lösungsprinzips dar und tragen jeweils weiter zur Erreichung der überraschenden Effekte und weiterer unerwarteten Vorteile bei, die nachfolgend beschrieben werden.

In der Beschreibung wird auf die beigefügten Zeichnungen verwiesen.

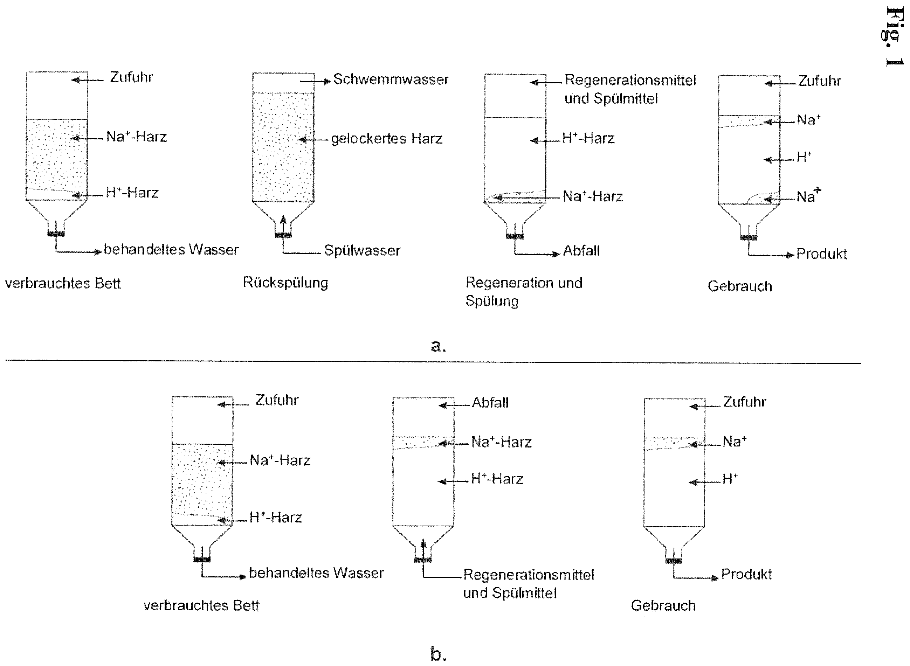

- Fig. 1a: stellt schematisch den Ablauf eines Gleichstromverfahrens zum Betrieb eines Ionenaustauschers dar.
- Fig. 1b: stellt schematisch den Ablauf eines Gegenstromverfahrens zum Betrieb eines Ionenaustauschers dar.

Erfindungsgemäß wird ein Absorber, insbesondere ein Superabsorber durch die Vernetzung eines Polymermaterials mit einem Sulfobernsteinsäurematerial erhalten.

Unter einem "Sulfobernsteinsäurematerial" wird dabei insbesondere eine wässrige Lösung der Sulfobernsteinsäure verstanden, vornehmlich eine wässrige Lösung mit 70 Gew.-% Sulfobernsteinsäure.

Es kann darunter aber auch eine wässrige Lösung mit einer anderen Sulfobernsteinsäurekonzentration oder eine Lösung von Sulfobernsteinsäure in einem von Wasser verschiedenen Lösungsmittel, beispielsweise einem Alkohol, verstanden werden.

Es kann darunter auch ein Derivat der Sulfobernsteinsäure, eine Mischung mehrerer Derivate der Sulfobernsteinsäure oder eine Mischung aus Sulfobernsteinsäure mit mindestens einem Derivat der Sulfobernsteinsäure oder eine Lösung von Sulfobernsteinsäure und/oder einem oder mehreren Sulfobernsteinsäurederivaten in einem geeigneten Lösungsmittel verstanden werden. Unter Derivaten werden dabei insbesondere das Sulfobernsteinsäureanhydrid, die Sulfobernsteinsäuremonochloride (HOOC-CH₂-CH(SO₃H)-COCl und HOOC-CH(SO₃H)-CH₂-COCl) und das Sulfobernsteinsäuredichlorid (ClOC-CH₂-CH(SO₃H)-COCl) verstanden.

Bei der Vernetzung des Polymermaterials durch ein Sulfobernsteinsäurematerial handelt es sich insbesondere um eine thermische Vernetzung. Andere Vernetzungsmechanismen, z.B. ein ionischer Vernetzungsmechanismus, können zu einem unerwünschten irreversiblen Kollabieren des Materials nach der Trocknung führen, was unerwünscht ist, da das entstehende Material dann kein oder nur ein sehr geringes Absorptionsvermögen aufweist.

Das erfindungsgemäß eingesetzte Sulfobernsteinsäurematerial ist frei oder im Wesentlichen frei von Alkalimetallionen.

Bevorzugt ist das eingesetzte Sulfobernsteinsäurematerial zusätzlich frei oder im Wesentlichen frei von Erdalkalimetallionen. Weiter bevorzugt ist das eingesetzte Sulfobernsteinsäurematerial zusätzlich frei oder im Wesentlichen frei von positiv geladenen Fremdionen jeder Art. Unter "Fremdionen" werden Ionen, insbesondere Metallionen verstanden, die nicht aufgrund einer im eingesetzten Sulfobernsteinsäurematerial stattfindenden Säure-Base-Gleichgewichtsreaktionen gebildet werden. So bilden sich in einer wässrigen Lösung von Sulfobernsteinsäure infolge der auftretenden Protolysereaktion Hydroniumionen; Hydroniumionen sind keine Fremdionen im Sinne der vorliegenden Erfindung. Wenn die eingesetzte Sulfobernsteinsäure beispielsweise teilweise in Form eines Natriumsalzes der Sulfobernsteinsäure vorläge bzw. ein anderes natriumionenhältiges Salz enthielte, würden die Natriumionen Fremdionen darstellen.

Eine Zusammensetzung wird als "im Wesentlichen" frei von dem vorstehend definierten Ion bzw. den vorstehend definierten Ionen bezeichnet, wenn der Gehalt an diesem Ion/diesen Ionen so gering ist, dass dieses Ion/diese Ionen im Wesentlichen keinen störenden Einfluss auf den Verlauf einer Umsetzung, der die Zusammensetzung unterzogen wird, und somit insbesondere in Bezug auf die Vernetzungsreaktion, hat/haben.

Es hat sich gezeigt, dass ein derartiger störender Einfluss insbesondere dann unterbleibt, wenn der Gehalt an Alkalimetallionen und gegebenenfalls der Gehalt an Erdalkalimetallionen und/oder anderer positiv geladener Fremdionen im Sulfobernsteinsäurematerial höchstens 500 ppm, bevorzugt höchstens 100 ppm, mehr bevorzugt höchstens 10 ppm ist.

Eine Zusammensetzung wird als frei von dem vorstehend definierten Ion bzw. den vorstehend definierten Ionen bezeichnet, wenn der Gehalt an diesem Ion/diesen Ionen so gering ist, dass dieses Ion/diese Ionen durch das zum Ionennachweis verwendete Verfahren nicht nachweisbar ist/sind. Die Nachweisgrenze für anorganische Kationen in einer Lösung liegt häufig bei 5 ppm.

Die jeweiligen Angaben des Gehalts an Alkalimetallionen, Erdalkalimetallionen bzw. anderen positiv geladenen Fremdionen in einem Sulfobernsteinsäurematerial werden nachfolgend auf das Gesamtgewicht des Sulfobernsteinsäurematerials bezogen, wobei die Einheit "ppm" als Gewichtskonzentrationsmaß verstanden wird. Folgendes Beispiel soll dies exemplarisch veranschaulichen: Handelt es sich bei dem Sulfobernsteinsäurematerial beispielsweise um eine Sulfobernsteinsäurelösung mit einem Sulfobernsteinsäuregehalt von 70 Gew.-%, so bedeutet ein Natriumionengehalt von 50 ppm, dass 1 kg dieser Sulfobernsteinsäurelösung 50 × 10⁻⁶ kg = 50 mg Natriumionen enthält.

Es ist im Rahmen der Erfindung bevorzugt, dass es sich bei dem Sulfobernsteinsäurematerial um eine wässrige Lösung von Sulfobernsteinsäure mit einem Sulfobernsteinsäuregehalt von 70 Gew.-% handelt. Wenn eine derartige Lösung beispielsweise einen Natriumionengehalt von 50 ppm aufweist, bedeutet dies, dass in der Lösung pro Kilogramm Sulfobernsteinsäure 50/0,7 = 71 mg Natriumionen vorhanden sind. Über das Molekulargewicht der Sulfobernsteinsäure von 198,15 g/mol wird berechnet, dass in einer derartigen Lösung pro Mol Sulfobernsteinsäure 71/(1000/198,15) = 14,15 mg (entsprechend 0,61 mmol) Natriumionen enthalten sind.

Vorzugsweise enthält das Polymermaterial, welches erfindungsgemäß vernetzt wird, Hydroxygruppen und/oder Aminogruppen.

Vorzugsweise enthält das Polymermaterial, welches erfindungsgemäß vernetzt wird, ionische Gruppen, die dem Absorber eine - vorzugsweise große oder sogar sehr große - Kapazität zur Absorption/Aufnahme/Bindung von Wasser und anderen polaren Flüssigkeiten verleihen. Bei diesen Gruppen handelt es sich beispielsweise um Carboxylatgruppen (-COO⁻). Entsprechend enthält das zu vernetzende Polymermaterial beispielsweise Hydroxygruppen und/oder Aminogruppen und optional zusätzlich Carboxygruppen und/oder Natriumcarboxylatgruppen und/oder Kaliumcarboxylatgruppen.

Im Rahmen der Erfindung ist es bevorzugt, dass das Polymermaterial ein Polymer umfasst, das aus der aus Polyvinylalkohol, Cellulose I, Cellulose II, Chitosan, Dextranen, Cyclodextrinen, Stärke, Alginaten, Carrageenen, Copolymeren der Acrylsäure und Natriumacrylaten bestehenden Gruppe ausgewählt ist.

Cellulose I, Cellulose II, Chitosan, Stärke, Alginate, Dextrane, Cyclodextrine und Carrageene sind dabei besonders leicht biologisch abbaubar. Daher können unter Verwendung dieser Polymere Absorber oder Superabsorber hergestellt werden, die besonders leicht biologisch abbaubar sind.

Unter Verwendung der Copolymere der Acrylsäure und Natriumacrylate lassen sich durch Vernetzung mit einem Sulfobernsteinsäurematerial Absorber oder Superabsorber herstellen, die eine besonders hohe Absorptionsfähigkeit aufweisen, d.h. die bezogen auf ihr Eigengewicht eine besonders große Menge an Wasser und anderen polaren Flüssigkeiten aufnehmen und zurückhalten können.

Polyvinylalkohol, Copolymere der Acrylsäure und Natriumacrylate sind an sich schwer oder gar nicht biologisch abbaubar. Durch den Einbau biologisch spaltbarer Einheiten ("Abbauschwachstellen") in die Polymerketten dieser Polymere kann deren biologische Abbaubarkeit verbessert werden bzw. überhaupt erst eine biologische Abbaubarkeit ermöglicht werden. Abbauschwachstellen können beispielsweise durch Copolymerisation geeignet ausgewählter Monomere erzeugt werden. Bei den Abbauschwachstellen kann es sich beispielsweise um funktionelle Gruppen, die hydrolytisch, oxidativ, photochemisch oder enzymatisch abgebaut werden können, handeln. Bei den Abbauschwachstellen kann es sich ferner beispielsweise um Kohlenstoff-Kohlenstoff-Doppelbindungen handeln.

Erfindungsgemäß wurde überraschend gefunden, dass die Vernetzungsreaktion des Polymermaterials nicht gestört wird, wenn darauf geachtet wird, dass das eingesetzte Sulfobernsteinsäurematerial frei oder im Wesentlichen frei von Alkalimetallionen, insbesondere frei oder im Wesentlichen frei von Alkalimetallionen und Erdalkalimetallionen sowie anderen positiv geladenen Fremdionen ist, so dass der erfindungsgemäße Absorber gut definierbar und einstellbar herzustellen ist, während gleichzeitig das Quellverhalten des mit diesem speziell eingestellten Sulfobernsteinsäurematerial hergestellten Absorbers in einer herkömmlich weder in Betracht gezogenen noch zu erwartenden Weise bestimmt werden kann. Außerdem können die genannten Ionen dann keinen störenden Einfluss auf Reaktionen der Sulfobernsteinsäure, insbesondere auf deren Vernetzungsreaktionen haben. Es hat sich gezeigt, dass die Vernetzungsreaktion durch die Einlagerung von Alkalimetallsalzen oder Salzen, die von anderen positiv geladenen Fremdionen mit Anionen gebildet werden, beispielsweise die Einlagerung von Natriumsalzen der der Sauerstoffsäuren des Schwefels (beispielsweise Natriumsulfat, Natriumhydrogensulfat, Natriumsulfit, Natriumhydrogensulfit) gestört wird. Erfindungsgemäß wird die Einlagerung solcher Salze vermieden.

Durch die erfindungsgemäße Verwendung eines derartigen Sulfobernsteinsäurematerials ist es beispielsweise möglich, dass durch die Zugabe von Sulfobernsteinsäurematerial zu einem Polymermaterial der Ionengehalt dieses Polymermaterials nicht oder nur unwesentlich durch den Eintrag von zusätzlichen, im Sulfobernsteinsäurematerial enthaltenen Alkalimetallionen und gegebenenfalls Erdalkalimetallionen und/oder anderen positiv geladenen Fremdionen verändert wird. Folglich ist es etwa möglich, eine Beeinflussung des Quellverhaltens eines unter Verwendung eines Sulfobernsteinsäurematerials hergestellten Absorbers oder Superabsorbers, die auf den Eintrag zusätzlicher Alkalimetallionen zurückzuführen ist, zu verringern oder überhaupt zu vermeiden.

Mittels geeigneter Messmethoden ist feststellbar, ob ein Absorber oder Superabsorber erfindungsgemäß, d.h. unter Verwendung eines Sulfobernsteinsäurematerials, welches frei oder im Wesentlichen frei von Alkalimetallionen und gegebenenfalls weiteren positiv geladenen Fremdionen ist, hergestellt ist. Dazu wird beispielsweise der Gehalt an Alkalimetallionen und gegebenenfalls weiteren positiv geladenen Fremdionen in einer aus einem Absorber oder Superabsorber bestehenden Probe gemessen, wozu die Probe zuvor bevorzugt thermisch aufgeschlossen wird. Der Gehalt der Probe an diesen Ionen kann beispielsweise mittels ICP-AAS gemessen werden. Der gemessene Gehalt an Alkalimetallionen und gegebenenfalls weiteren positiv geladenen Fremdionen wird mit dem Gehalt, welchen das Ausgangspolymermaterial an diesen Ionen aufweist, verglichen. Anstatt der direkten Messung der Konzentration an Alkalimetallionen und anderen positiv geladenen Fremdionen kann im Falle von Absorbern oder Superabsorbern, die unter Verwendung von Carboxygruppen- und Carboxylatgruppen enthaltenden Polymeren hergestellt worden sind, auch eine pH-Wert-Messung vorgenommen werden, da der pH-Wert einer wässrigen Flüssigkeit, die mit einem solchen Absorber oder Superabsorber in Kontakt steht, vom Verhältnis zwischen im Absorber oder Superabsorber vorhandenen Carboxygruppen und Carboxylatgruppen abhängt.

Erfindungsgemäß ist die Verwendung eines derartigen Sulfobernsteinsäurematerials als Quervernetzer und/oder als Oberflächenvernetzer zur Herstellung eines Absorbers vorgesehen.

Ein Vorteil der Erfindung besteht beispielsweise darin, die Eigenschaften des Absorbers oder Superabsorbers, insbesondere die Absorptionsfähigkeit und die Kinetik des Quellvorgangs, genau einstellen zu können. Entsprechend ist es möglich, Absorber oder Superabsorber mit reproduzierbaren Eigenschaften und aus diesen Absorbern oder Superabsorbern wiederum Produkte, die eine hochgradige Homogenität (innerhalb ein und desselben Produkts sowie bei Vergleich von Produkten derselben Produktionscharge und von Produkten verschiedener Produktionschargen) aufweisen, bereitzustellen. Entsprechend ist es also möglich, mit diesen Absorbern oder Superabsorbern Produkte mit hoher und gleichbleibender Qualität bereitzustellen.

Ein weiterer Vorteil des erfindungsgemäßen Absorbers oder Superabsorbers besteht beispielsweise darin, dass Sulfobernsteinsäure selbst bzw. Sulfosuccinylgruppen als integraler Bestandteil im mit einem Sulfobernsteinsäure quervernetzten Polymer leicht biologisch abbaubar sind. Dadurch ist eine zumindest teilweise biologische Abbaubarkeit des Absorbers oder Superabsorbers gegeben, ohne dass an das Milieu, in dem der Abbau erfolgen soll, besondere Anforderungen zu stellen sind. So ist es möglich, unter Verwendung des erfindungsgemäßen Absorbers oder Superabsorbers Produkte bereitzustellen, die schneller als in herkömmlicher Weise vernetzte Absorber oder Superabsorber abgebaut werden, nachdem sie in die Umwelt (Boden, Gewässer usw.) gelangt sind, und daher nicht zu anhaltender Verschmutzung der Umwelt führen.

Es ist dabei besonders vorteilhaft, dass der biologische Abbau von Sulfobernsteinsäure rasch erfolgt. Dadurch kann der biologische Abbau des erfindungsgemäßen Absorbers oder Superabsorbers im Vergleich zu einem unter Verwendung eines anderen Vernetzers hergestellten Absorbers oder Superabsorbers beschleunigt werden.

Bevorzugt ist der erfindungsgemäße Absorber oder Superabsorber vollständig biologisch abbaubar. Dies kann dadurch erreicht werden, dass das zu dessen Herstellung eingesetzte und der Vernetzung mit einem Sulfobernsteinsäurematerial unterzogene Polymermaterial seinerseits biologisch abbaubar ist.

Ein weiterer Vorteil der Erfindung ergibt sich im Falle von Absorbern und Superabsorbern, die durch Vernetzung eines Polymermaterials, das neben Hydroxygruppen (-OH) auch Carboxygruppen (-COOH) und Carboxylatgruppen (-COO⁻) enthält, erhältlich sind. Die Anwesenheit von Carboxylatgruppen (-COO⁻) im Polymer bedingt die Anwesenheit von Kationen, bei denen es sich insbesondere um Alkalimetallionen handelt, zum Ladungsausgleich. Das Quellverhalten eines durch Quervernetzung eines derartigen Polymermaterials unter Verwendung des speziell bereitgestellten bzw. vorbereiteten Sulfobernsteinsäurematerials hergestellten Absorbers wird erfindungsgemäß praktisch nur noch durch die gut definierbare Menge an ionischen Bestandteilen, welche in der Polymermatrix gebunden sind, bestimmt und nicht durch einen unkontrollierbaren Eintrag durch das Sulfobernsteinsäurematerial beeinträchtigt. Für das Quellverhalten maßgeblich ist somit allein das Verhältnis zwischen der Anzahl an Carboxygruppen (-COOH) einerseits und der Anzahl an Carboxylatgruppen, die die Anwesenheit beispielsweise eines Alkalimetallions X⁺ zum Ladungsausgleich bedingen, andererseits (-COO⁻X⁺), d.h. das Verhältnis -COO⁻X⁺ :-COOH im Polymermaterial. Um die Gesamtmenge an einwertigen Alkalimetallionen beim Herstellen von Absorbern oder Superabsorbern genau einstellen zu können, ist es nötig und erfindungsgemäß möglich, deren Gehalt in den bei der Herstellung verwendeten Ausgangsstoffen zu kennen und zu definieren. Erfindungsgemäß können somit Polymerketten mit definierter Ionenkonzentration eingesetzt und mit Quervernetzern behandelt werden, um initiale Körnchen des Absorbers oder Superabsorbers zu formen, d.h. um aus den meist wasserlöslichen Polymeren durch Quervernetzung einen wasserunlöslichen Absorber oder Superabsorber mit körniger Struktur herzustellen. Optional können die initialen Körnchen durch eine Oberflächenvernetzung, d.h. eine weitere Vernetzung in oberflächennahen Bereichen des Körnchens, nachbehandelt werden. Durch Auswahl und Einstellung des Quervernetzers gemäß der Erfindung, der den Ionengehalt des hergestellten Absorbers oder Superabsorbers nicht oder nur in geringem Maße verändert, ist es möglich, die Gesamtmenge an einwertigen Ionen genau einzustellen und Absorber oder Superabsorber hoher Qualität und hoher Reproduzierbarkeit bereitzustellen.

Die mittlere Korngröße des Absorbers (d50-Wert) oder Superabsorbers wird mittels Laserbeugung oder mittels Sieben bestimmt. In geeigneter Weise kommt dabei Laserbeugung zum Einsatz, wenn die mittlere Korngröße kleiner als 500 µm ist, und Sieben, wenn die mittlere Korngröße größer als 500 µm ist.

Darüber hinaus ist bei einem hohen Gehalt an Alkalimetall- und/oder anderen positiv geladenen Fremdionen, insbesondere an Alkalimetallionen und vor allem an Natriumionen, die Quervernetzungsreaktion gestört. Beispielsweise kann bei Anwesenheit derartiger Ionen die Geschwindigkeit der Quervernetzungsreaktion beträchtlich herabgesetzt sein. Durch die erfindungsgemäße Verwendung des Sulfobernsteinsäurematerials, das frei oder im Wesentlichen frei von Natriumionen und bevorzugt weiteren Alkalimetallionen und/oder Erdalkalimetallionen und/oder anderen positiv geladenen Fremdionen ist, ist es möglich, zu verhindern, dass der Gehalt an Ionen ein für den gewünschten Verlauf der Quervernetzungsreaktion akzeptables Maß überschreitet. Beispielsweise können zwei- oder höherwertige Ionen mit dem Polymermaterial ein dreidimensionales ionisches Gelnetzwerk ausbilden, wodurch die gewünschte kovalente Vernetzung unterdrückt wird. Zum Beispiel gelieren Alginate bei Anwesenheit von Calciumionen. Bei Trocknung kollabieren diese Netzwerke jedoch irreversibel und zeigen nur mehr ein sehr geringes bzw. kein Quellverhalten.

Gemäß einer bevorzugten Ausführungsform der Erfindung wird das Sulfobernsteinsäurematerial, das frei oder im Wesentlichen frei von Alkalimetallionen, Erdalkalimetallionen und anderen positiv geladenen Fremdionen ist, hergestellt, indem eine geeignete Ausgangsverbindung, die aus der aus Maleinsäureanhydrid, Maleinsäure, Fumarsäure sowie Derivaten davon bestehenden Gruppe ausgewählt sein kann, mit Schwefeldioxid in wässriger Lösung zur Reaktion gebracht wird. Es ist dabei möglich, dass im Zuge der Herstellung des Sulfobernsteinsäurematerials intermediär das Anhydrid der Sulfobernsteinsäure oder ihrer Derivate gebildet wird. Die auf diese Weise bereitgestellte wässrige Sulfobernsteinsäurelösung ist frei oder im Wesentlichen frei von jeglichen Alkalimetallionen und/oder Erdalkalimetallionen und/oder anderen positiv geladenen Fremdionen.. Unter "Derivaten" werden dabei insbesondere die Monoester und Diester der Maleinsäure und der Fumarsäure mit Alkoholen, bevorzugt mit aliphatischen Alkoholen, mehr bevorzugt mit aliphatischen Alkoholen der Formel CₙH₂ₙ₊₁OH mit n = 1, 2, 3 oder 4 , verstanden. Zur Verseifung von unter Einsatz derartiger Ester als Ausgangsstoffe hergestellter Sulfobernsteinsäureester wird bevorzugt ein basischer Ionenaustauscher verwendet, da auf diese Weise die Verwendung einer Base, welche positiv geladene Fremdionen enthält, vermieden werden kann. Unter "Derivaten" können beispielsweise auch die Säurechloride der Maleinsäure und der Fumarsäure (Monochloride HOOC-CH₂=CH₂COCl und Dichloride ClOC-CH₂=CH₂-COCl) sowie Maleinsäureanhydrid verstanden werden.

Gemäß einer weiteren bevorzugten Ausführungsform der Erfindung wird ein Sulfobernsteinsäureausgangsmaterial, das zunächst zumindest Alkalimetallionen und optional zusätzlich Erdalkalimetallionen und/oder andere positiv geladene Fremdionen enthalten hat, mittels eines stark sauren Ionenaustauschers, bei welchem es sich um einen Gegenstromaustauscher (d.h. um einen Ionenaustauscher, der im Gegenstromverfahren betrieben wird) handelt, entsalzt, um das Sulfobernsteinsäurematerial bereitzustellen, das frei oder im Wesentlichen frei von den genannten Ionen ist. Durch die Anwendung des Gegenstromverfahrens ist es bei vergleichsweise niedrigem Zeit- und Kostenaufwand möglich, durch Ionenaustausch einen im Vergleich zur Anwendung eines Gleichstromverfahrens höheren Entsalzungsgrad des Sulfobernsteinsäurematerials zu erreichen.

Gemäß einer weiteren bevorzugten Ausführungsform der Erfindung wird ein Sulfobernsteinsäureausgangsmaterial, das Alkalimetallionen und optional Erdalkalimetallionen und/oder andere positiv geladene Fremdionen enthalten hat, mittels einer für Alkalimetallionen durchlässigen Membran entsalzt, um das Sulfobernsteinsäurematerial bereitzustellen, das frei oder im Wesentlichen frei von den genannten Ionen ist.

Gemäß einer weiteren bevorzugten Ausführungsform der Erfindung wird ein Sulfobernsteinsäureausgangsmaterial, das zunächst Alkalimetallionen und optional Erdalkalimetallionen und/oder andere positiv geladene Fremdionen enthalten hat, durch eine Kombination aus einem stark sauren Ionenaustauscher, bei welchem es sich bevorzugt um einen Gegenstromaustauscher handelt, und einer für zumindest Alkalimetallionen durchlässigen Membran entsalzt, um das Sulfobernsteinsäurematerial bereitzustellen, die frei oder im Wesentlichen frei von den genannten Ionen ist.

Erfindungsgemäß ist ein Absorber durch die Vernetzung eines Polymermaterials mit einem Sulfobernsteinsäurematerial erhältlich.

Gemäß einer weiteren bevorzugten Ausführungsform der Erfindung handelt es sich bei dem Absorber um einen Superabsorber.

Gemäß einer weiteren bevorzugten Ausführungsform der Erfindung ist das Sulfobernsteinsäurematerial frei oder im Wesentlichen frei von Alkalimetallionen.

Gemäß einer weiteren bevorzugten Ausführungsform der Erfindung ist dabei der Gehalt an Alkalimetallionen im Sulfobernsteinsäurematerial höchstens 500 ppm, bevorzugt höchstens 100 ppm, mehr bevorzugt höchstens 10 ppm.

Gemäß einer weiteren bevorzugten Ausführungsform der Erfindung ist das Sulfobernsteinsäurematerial frei oder im Wesentlichen frei von Alkalimetallionen und zusätzlich frei oder im Wesentlichen frei von Erdalkalimetallionen.

Gemäß einer weiteren bevorzugten Ausführungsform der Erfindung ist das Sulfobernsteinsäurematerial frei oder im Wesentlichen frei von Alkalimetallionen und zusätzlich anderen positiv geladenen Fremdionen.

Gemäß einer weiteren bevorzugten Ausführungsform der Erfindung ist der Gehalt an Alkalimetallionen und Erdalkalimetallionen im Sulfobernsteinsäurematerial insgesamt höchstens 500 ppm, bevorzugt höchstens 100 ppm, mehr bevorzugt höchstens 10 ppm.

Gemäß einer weiteren bevorzugten Ausführungsform der Erfindung ist der Gehalt an positiv geladenen Fremdionen im Sulfobernsteinsäurematerial insgesamt höchstens 500 ppm, bevorzugt höchstens 100 ppm, mehr bevorzugt höchstens 10 ppm.

Gemäß einer weiteren bevorzugten Ausführungsform der Erfindung umfasst das Sulfobernsteinsäurematerial mindestens eine der aus der aus Sulfobernsteinsäure, Sulfobernsteinsäureanhydid, den Sulfobernsteinsäuremonochloriden und Sulfobernsteinsäuredichlorid bestehenden Gruppe ausgewählte Verbindung. Dabei umfasst das Sulfobernsteinsäurematerial bevorzugt Sulfobernsteinsäure. Dabei ist das Sulfobernsteinsäurematerial mehr bevorzugt eine wässrige Lösung von Sulfobernsteinsäure, noch mehr bevorzugt eine wässrige Lösung von Sulfobernsteinsäure mit einem Sulfobernsteinsäuregehalt von 65 bis 75 Gew.-%, insbesondere bevorzugt eine wässrige Lösung von Sulfobernsteinsäure mit einem Sulfobernsteinsäuregehalt von 70 Gew.-%.

Gemäß einer weiteren bevorzugten Ausführungsform der Erfindung ist die Vernetzung eine Kernvernetzung und/oder eine Oberflächenvernetzung .

Gemäß einer weiteren bevorzugten Ausführungsform der Erfindung ist der Absorber oder Superabsorber biologisch abbaubar, insbesondere kompostierbar.

Gemäß einer weiteren bevorzugten Ausführungsform der Erfindung ist das Polymermaterial zumindest teilweise eine Polymerstruktur, welche Abbauschwachstellen, insbesondere Kohlenstoff-Kohlenstoff-Doppelbindungen, aufweist und welche insbesondere eine lineare Polymerstruktur aufweist.

Gemäß einer weiteren bevorzugten Ausführungsform der Erfindung enthält das Polymermaterial Hydroxygruppen.

Gemäß einer weiteren bevorzugten Ausführungsform der Erfindung enthält das Polymermaterial Aminogruppen zusätzlich oder alternativ zu den Hydroxygruppen.

Gemäß einer weiteren bevorzugten Ausführungsform der Erfindung enthält das Polymermaterial mindestens ein aus der aus Polyvinylalkohol, Cellulose I, Cellulose II, Chitosan, Stärke, Alginaten, Carrageenen, Dextranen, Cycodextrinen, Copolymeren der Acrylsäure und Natriumacrylaten bestehenden Gruppe ausgewähltes Polymer.

Gemäß einer weiteren bevorzugten Ausführungsform der Erfindung weist das Polymermaterial Carboxygruppen und/oder Natriumcarboxylatgruppen und/oder Kaliumcarboxylatgruppen auf.

Gemäß einer weiteren bevorzugten Ausführungsform der Erfindung kann der Absorber oder Superabsorber bei Kontakt mit einer polaren Flüssigkeit mindestens sein halbes, bevorzugt mindestens sein einfaches, mehr bevorzugt mindestens sein doppeltes Eigengewicht an der polaren Flüssigkeit aufnehmen. Bei der polaren Flüssigkeit handelt es sich bevorzugt um eine wässrige Flüssigkeit, mehr bevorzugt um eine wässrige Lösung, noch mehr bevorzugt um Wasser, insbesondere um deionisiertes Wasser.

Gemäß einer weiteren bevorzugten Ausführungsform der Erfindung liegt der Absorber oder Superabsorber zumindest teilweise in Form von Körnchen vor, wobei die Körnchen bevorzugt eine mittlere Korngröße (d50) von mindestens 10 µm, mehr bevorzugt mindestens 20 µm, noch mehr bevorzugt mindestens 50 µm aufweisen.

Gemäß einer weiteren bevorzugten Ausführungsform der Erfindung liegt der Absorber oder Superabsorber zumindest teilweise in Form von Körnchen vor, wobei die Körnchen bevorzugt eine mittlere Korngröße (d50) von höchstens 4000 µm, mehr bevorzugt höchstens 2000 µm, noch mehr bevorzugt höchstens 1000 µm aufweisen.

Gemäß einer weiteren bevorzugten Ausführungsform der Erfindung wird ein erfindungsgemäßer Absorber oder Superabsorber in einem der aus der aus Schäumen, Formkörpern, Fasern, Folien, Filmen, Kabeln, Dichtungsmaterialien, flüssigkeitsaufnehmenden Hygieneartikeln, Trägern für pflanzen- und pilzwachstumsregulierende Mittel, Wasserspeichergranulaten für die Verwendung im Agrarsektor, gelbildenden Löschmitteln, Verpackungszusätzen, Baustoffen, Damenhygieneprodukten, Babyhygieneprodukten, Tierhygieneprodukten, Wundauflagen, Erwachseneninlcontinenzprodukten, für chromatographische Trennungen verwendbaren Absorbermaterialien (beispielsweise Säulenmaterialien), Trägermaterialien für Enzyme und/oder Mikroorganismen, für Membrantrennungen verwendbaren Materialien sowie im Körperpflege- und/oder Kosmetikbereich einsetzbaren Absorbermaterialien bestehenden Gruppe ausgewählten Produkt verwendet.

Gemäß einer weiteren bevorzugten Ausführungsform der Erfindung wird ein Sulfobernsteinsäurematerial für die Herstellung eines Absorbers oder Superabsorbers durch eine Vernetzung, insbesondere eine thermische Vernetzung, eines Polymermaterials mit dem Sulfobernsteinsäurematerial verwendet. Bevorzugt ist das Sulfobernsteinsäurematerial frei oder im Wesentlichen frei von Alkalimetallionen.

Bei dem Verfahren gemäß einer weiteren bevorzugten Ausführungsform der Erfindung handelt es sich um ein Verfahren zum Herstellen eines Absorbers oder Superabsorbers durch eine Vernetzung, insbesondere eine thermische Vernetzung, eines Polymermaterials mit einem Sulfobernsteinsäurematerial, wobei das Sulfobernsteinsäurematerial frei oder im Wesentlichen frei von Alkalimetallionen ist, wobei das Verfahren bevorzugt zumindest folgende Schritte umfasst:
(a) Bereitstellen des Polymermaterials, das optional zumindest teilweise in einem Lösungsmittel gelöst ist,
   wobei es sich bei dem Lösungsmittel insbesondere um Wasser, bevorzugt um deionisiertes Wasser, handelt;
(b) Mischen des Polymermaterials mit dem Sulfobernsteinsäurematerial,
   wobei das Sulfobernsteinsäurematerial frei oder im Wesentlichen frei von Alkalimetallionen, bevorzugt frei oder im Wesentlichen frei von Alkalimetall- und Erdalkalimetallionen, mehr bevorzugt frei oder im Wesentlichen frei von positiv geladenen Fremdionen ist und
   wobei das Sulfobernsteinsäurematerial optional zumindest teilweise als Lösung vorliegt,
   wobei es sich bei der Lösung insbesondere um eine Lösung in Wasser oder in einer Mischung aus Aceton und Wasser, bevorzugt um eine Lösung in deionisiertem Wasser, handelt;
(c) optional fortgesetztes mechanisches Behandeln der Mischung, insbesondere Rühren;
(d) optional Trocknen, insbesondere bei erhöhter Temperatur und/oder vermindertem Druck;
(e) Durchführen einer Temperaturbehandlung zum Vernetzen des Polymermaterials mit der Sulfobernsteinsäure.

Gemäß einer weiteren bevorzugten Ausführungsform der Erfindung wird diese Temperaturbehandlung zumindest zeitweise bei einer Temperatur von mindestens 80 °C, bevorzugt mindestens 100 °C, noch mehr bevorzugt mindestens 110 °C durchgeführt.

Gemäß einer weiteren bevorzugten Ausführungsform der Erfindung wird diese Temperaturbehandlung zumindest zeitweise bei einer Temperatur von höchstens 160 °C, bevorzugt höchstens 140 °C, mehr bevorzugt höchstens 130 °C durchgeführt.

Gemäß einer weiteren bevorzugten Ausführungsform der Erfindung wird diese Temperaturbehandlung zumindest zeitweise bei einer Temperatur von etwa 120 °C durchgeführt.

Gemäß einer weiteren bevorzugten Ausführungsform der Erfindung beträgt das Gewichtsverhältnis der Sulfobernsteinsäure oder des Sulfobernsteinsäurederivats und des Polymermaterials in dem Verfahren zum Herstellen eines Absorbers oder Superabsorbers mindestens 0,01:1, bevorzugt mindestens 0,02:1, mehr bevorzugt mindestens 0,05:1, noch mehr bevorzugt mindestens 0,1:1 und/oder höchstens 5:1, bevorzugt höchstens 3:1, mehr bevorzugt höchstens 1:1, noch mehr bevorzugt höchstens 0,5:1, insbesondere bevorzugt höchstens 0,1:1.

Bei dem Verfahren gemäß einer weiteren bevorzugten Ausführungsform der Erfindung handelt es sich um ein Verfahren zum Herstellen eines Sulfobernsteinsäurematerials umfassend mindestens eine aus der aus Sulfobernsteinsäure und den Monoestern und Diestern der Sulfobernsteinsäure bestehenden Gruppe ausgewählten Verbindung, bevorzugt einer Lösung der mindestens einen Verbindung, insbesondere einer wässrigen Lösung der mindestens einen Verbindung, wobei das Verfahren zumindest folgende Schritte umfasst:
(a) Umsetzen mindestens einer aus der aus Maleinsäureanhydrid, Maleinsäure, Fumarsäure, Monoestern und Diestern der Maleinsäure sowie Monoestern und Diestern der Fumarsäure bestehenden Gruppe ausgewählten Verbindung mit mindestens einem aus der aus Alkalimetalldisulfit, Erdalkalimetalldisulfit, Alkalimetallsulfit, Erdalkalimetallsulfit, Alkalimetallhydrogensulfit und Erdalkalimetallhydrogensulfit bestehenden Gruppe ausgewählten Reaktanden;
(b) optional Verseifung mittels einer Lauge oder einem stark basischen Ionenaustauscher, falls im Schritt des Umsetzens ein Ester der Maleinsäure und/oder der Fumarsäure verwendet worden ist;
(c) Entsalzung mittels eines Ionenaustauschers im Gegenstromverfahren und/oder mittels eines Membrantrennverfahrens, insbesondere eines Nanofiltrationsverfahrens.

Dabei handelt es sich bei den eingesetzten Monoestern und Diestern bzw. den erhaltenen Monoestern und Diestern der Sulfobernsteinsäure bevorzugt um Monoester und Diester, die die entsprechenden Säuren mit aliphatischen Alkoholen der Formel CₙH₂ₙ₊₁OH mit n = 1, 2, 3 oder 4 bilden können.

Bei dem Verfahren gemäß einer weiteren bevorzugten Ausführungsform der Erfindung handelt es sich um ein Verfahren zum Herstellen eines Sulfobernsteinsäurematerials umfassend mindestens eine aus der aus Sulfobernsteinsäure und den Monoestern und Diestern, die Sulfobernsteinsäure bestehenden Gruppe ausgewählten Verbindung, bevorzugt einer Lösung der mindestens einen Verbindung, insbesondere einer wässrigen Lösung der mindestens einen Verbindung,
wobei mindestens eine aus der aus Maleinsäureanhydrid, Maleinsäure, Fumarsäure, Monoester und Diestern der Maleinsäure sowie Monoester und Diester Estern der Fumarsäure bestehenden Gruppe ausgewählte Verbindung mit Schwefeldioxid zur Sulfobernsteinsäure umgesetzt wird.

Dabei handelt es sich bei den eingesetzten Monoestern und Diestern bzw. den erhaltenen Monoestern und Diestern der Sulfobernsteinsäure bevorzugt um Monoester und Diester, die die entsprechenden Säuren mit aliphatischen Alkoholen der Formel CₙH₂ₙ₊₁OH mit n = 1, 2, 3 oder 4 bilden können.

Gemäß einer bevorzugten Ausführungsform der Erfindung wird die Umsetzung in einer wässrigen Reaktionsmischung, insbesondere in einer wässrigen Reaktionslösung durchgeführt, in die Schwefeldioxid unter Überdruck eingeleitet wird.

Gemäß einer weiteren bevorzugten Ausführungsform der Erfindung beträgt für die Umsetzung das Verhältnis der Stoffmenge des eingesetzten Schwefeldioxids und der Stoffmenge der eingesetzten aus Maleinsäureanhydrid, Maleinsäure, Fumarsäure, Monoestern und Diestern der Maleinsäure sowie Monoestern und Diestern der Fumarsäure bestehenden Gruppe ausgewählten Verbindung höchstens 1000, bevorzugt höchstens 500, mehr bevorzugt höchstens 100.

Gemäß einer weiteren bevorzugten Ausführungsform der Erfindung beträgt für die Umsetzung das Verhältnis der Stoffmenge des eingesetzten Schwefeldioxids und der Stoffmenge der eingesetzten aus Maleinsäureanhydrid, Maleinsäure, Fumarsäure, Monoestern und Diestern der Maleinsäure sowie Monoestern und Diestern der Fumarsäure bestehenden Gruppe ausgewählten Verbindung mindestens 1, bevorzugt mindestens 5, mehr bevorzugt mindestens 10.

Gemäß einer weiteren bevorzugten Ausführungsform der Erfindung beträgt die Temperatur der Reaktionsmischung bei der Umsetzung mindestens 0 °C, bevorzugt mindestens 10 °C, mehr bevorzugt mindestens 20 °C.
Gemäß einer weiteren bevorzugten Ausführungsform der Erfindung beträgt die Temperatur der Reaktionsmischung bei der Umsetzung höchstens 250 °C, bevorzugt höchstens 200 °C, mehr bevorzugt höchstens 190 °C.

Durch den Einsatz von alkalifreiem oder im Wesentlichen alkalifreiem Sulfobernsteinsäurematerial konnte ein Quervernetzer gefunden werden, der nicht nur biologisch abbaubar, sondern auch für die Herstellung eines Absorbers oder Superabsorbers mit definiertem Quellverhalten geeignet ist. Dies wird erfindungsgemäß durch das neue Verfahren zur Herstellung von Sulfobernsteinsäurematerial unter Einsatz von Schwefeldioxid oder durch die besonders ausgewählten Verfahren zur Entsalzung eines Sulfobernsteinsäurematerials ermöglicht.

Verfahren zur Umsetzung von Maleinsäure, Maleinsäureanhydrid oder Fumarsäure bzw. deren Derivaten (beispielsweise Ester der genannten Säuren) in wässrigem Milieu mit Sulfitsalzen (Sulfite, Hydrogensulfite, Disulfite von Metallen) sind im Stand der Technik bekannt.

Die DE 25 07 520 A1 offenbart ein Verfahren, in welchem ein Maleinsäuremonoalkylester mit Natriumsulfit zur Reaktion gebracht und der entsprechende Natriumsulfosuccinatmonoester erhalten wird.

Die US 5,543,555 A offenbart ein Verfahren, in welchem ein Maleinsäurediester mit einer Mischung aus Natriumdisulfit und Natriumsulfit in wässrigem Milieu zur Reaktion gebracht und der entsprechende Natriumsulfosuccinatdiester erhalten wird.

Die JP 2010064987 A offenbart ein Verfahren in welchem Fumarsäure mit unterschiedlichen Mischungen von Natriumdisulfit und Natriumsulfit zur Reaktion gebracht und das entsprechende Natriumsulfosuccinat erhalten wird.

Die Reaktionsprodukte dieser bekannten Verfahren, d.h. Sulfobernsteinsäure bzw. die entsprechenden Sulfobernsteinsäurederivate, fallen jedoch als Salze mit Metallkationen, insbesondere als Alkalimetall- oder Erdalkalimetallsalze an.

Um in weiterer Folge die freie Säureform der Sulfobernsteinsäure bzw. der entsprechenden Derivate, beispielsweise Ester, zu erhalten, wird durch zusätzliche Reinigungsschritte wie durch Einsatz eines Ionenaustauschers das Alkalimetallion (bzw. bei Einsatz von Sulfitsalzen anderer Metalle die entsprechenden Kationen) gegen Wasserstoff ausgetauscht. Als separater Reaktionsschritt wird in der JP 2010064987 A die Herstellung der freien Säureform der Sulfobernsteinsäure ausgehend von Natriumsulfosuccinat durch die Verwendung von sauren Ionenaustauschern beschrieben. Die JP 2010064987 A lässt dabei offen, ob die Ionenaustauscher im Gleichstrom- oder Gegenstromverfahren betrieben werden.

Der im Stand der Technik bekannte Einsatz von Ionenaustauschern ist zeitintensiv und erfordert große Mengen an Lösemitteln zur Regeneration und Konditionierung. Die Entsorgung der dabei entstehenden Abfälle (beispielsweise Abwässer) ist außerdem ressourcen- und kostenintensiv.

Wie weiter oben ausgeführt ist es für die Herstellung effizienter biologisch abbaubarer Absorberprodukte oder Superabsorberprodukte wichtig, einen Quervernetzer, der frei oder im Wesentliche frei von Alkalimetallionen ist, zur Verfügung zu haben, um beispielsweise die Quellung des Endprodukts genau zu definieren, d.h. das Quellverhalten eines Absorbers oder Superabsorbers im Zuge der Herstellung desselben vorauszubestimmen.

Überraschenderweise kann dann ein Sulfobernsteinsäurematerial, das frei oder im Wesentlichen frei von Alkalimetallionen ist, als ein solcher Quervernetzer eingesetzt werden.

Sulfobernsteinsäurematerial, das frei oder im Wesentlichen frei von Alkalimetallionen ist, kann auf besonders effiziente und ökonomische Weise hergestellt werden, wenn dabei im Gegensatz zum genannten Stand der Technik die Verwendung von Alkalimetallsulfitsalzen wie beispielsweise Natriumsulfit, Natriumhydrogensulfit und Natriumdisulfit, Erdalkalimentallsulfitsalzen und Sulfitsalzen anderer Metalle vermieden wird. In diesem Fall werden keine Alkalimetallionen, Erdalkalimetallionen und andere Metallionen eingebracht. Die Sulfobernsteinsäure wird in der gewünschten freien Säureform erhalten, ohne dass dafür ein zusätzlicher Ionenaustauschprozess nötig ist.

Die Erfinder haben überraschend gefunden, dass Sulfobernsteinsäure durch Reaktion mindestens einer der aus der aus Maleinsäure, Maleinsäureanhydrid, Fumarsäure und deren Derivaten bestehenden Gruppe ausgewählten Verbindung mit Schwefeldioxid bei Anwesenheit von Wasser hergestellt werden kann.

Unter Derivaten werden dabei insbesondere die Monoester und Diester, die die Maleinsäure und die Fumarsäure mit Alkoholen, bevorzugt mit aliphatischen Alkoholen, mehr bevorzugt mit aliphatischen Alkoholen der Formel CₙH₂ₙ₊₁OH mit n = 1, 2, 3 oder 4 bilden, verstanden.

Ein besonderer Vorteil des erfindungsgemäßen Verfahrens zur Herstellung von für die Verwendung zum Herstellen von Absorbern oder Superabsorbern vorgesehener Sulfobernsteinsäure unter Verwendung von Schwefeldioxid anstelle von Sulfitsalzen ist die Möglichkeit der völligen Abwesenheit von Alkalimetallen, Erdalkalimetallen und anderen positiv geladenen Fremdionen im Reaktionsprozess und damit einhergehend die Entbehrlichkeit eines zusätzlichen Ionenaustauschschritts.

In einer bevorzugten Ausführungsform dieses Verfahrens wird in eine mit Schwefeldioxid gesättigte wässrige Phase Maleinsäure, Maleinsäureanhydrid, Fumarsäure oder eine Mischung aus mindestens zwei dieser Stoffe eingetragen. Maleinsäureanhydrid kann dabei beispielsweise als Feststoff oder in flüssiger Form als Schmelze zugegeben werden. Alternativ oder zusätzlich kann eine unter Verwendung von Maleinsäure, Maleinsäureanhydrid, Fumarsäure oder einer Mischung aus mindestens zwei dieser Stoffe hergestellte wässrige Lösung zugegeben werden. Es wird Schwefeldioxid in das Reaktionsgemisch eingetragen. Der Reaktionsverlauf wird bevorzugt dünnschichtchromatographisch kontrolliert. Bevorzugt wird so lange Schwefeldioxid in das Reaktionsgemisch eingetragen, bis die dünnschichtchromatographische Analyse kein Edukt mehr anzeigt. Der Eintrag von Schwefeldioxid in die Reaktionslösung erfolgt bevorzugt unter Druck. Nach Beendigung der Reaktion wird das Reaktionsgefäß entspannt und die Reaktionslösung an der Atmosphäre erhitzt, um überschüssiges gelöstes Schwefeldioxid auszutreiben. Die erhaltene wässrige Lösung von Sulfobernsteinsäure ist völlig frei von jeglichen Alkalimetallionen, Erdalkalimetallionen und anderen positiv geladenen Fremdionen.

Gemäß einer weiteren bevorzugten Ausführungsform dieses Verfahrens werden gasförmiges Schwefeldioxid und Wasserdampf unter Druck in eine Schmelze von Maleinsäureanhydrid eingetragen, um das Maleinsäureanhydrid zur Sulfobernsteinsäure umzusetzen.

Die Entsalzung von unter Verwendung von Sulfitsalzen hergestellter Sulfobernsteinsäure mit Hilfe von stark sauren Kationenaustauschern im Gleichstromverfahren ist hingegen sehr zeit- und ressourcenintensiv und daher ökonomisch nachteilig.

Gemäß einer Ausführungsform der Erfindung werden Kationenaustauscher im Gegenstromverfahren, in dem der Ionenaustauscher im Gegenstrom regeneriert wird, verwendet. Dadurch kann die Entsalzung bei geringem Zeit- und Ressourcenaufwand durchgeführt werden.

In Fig. 1a und Fig. 1b sind das Gleichstromverfahren und das Gegenstomverfahren zum Betrieb eines sauren Ionenaustauschers schematisch gegenübergestellt. In der Darstellung dieser Zeichnungen sind Natriumionen (Na⁺) lediglich beispielhaft als die durch Ionenaustausch aus einer Lösung zu entfernenden Ionen angegeben. Es kann sich bei diesen Ionen alternativ oder zusätzlich um von Natriumionen verschiedene Alkalimetallionen, Erdalkalimetallionen oder andere positiv geladene Ionen (mit der Ausnahme von H⁺) handeln.

Im Falle des Gleichstromverfahrens (Fig. 1a) erfolgt die Regeneration des verbrauchten Ionenaustauscherharzes mittels eines Regenerationsmittels, das in derselben Richtung durch den Ionenaustauscher fließt, in der auch die dem Ionenaustausch zu unterziehende Lösung beim Gebrauch des Ionenaustauschers hindurchfließt. In den Zeichnungen der Fig. 1 a ist das die Richtung von oben nach unten. Die Regeneration ist in Fig. 1a in der dritten Zeichnung von links dargestellt.

Im Falle des Gegenstromverfahrens (Fig. 1b) erfolgt die Regeneration des verbrauchten Ionenaustauscherharzes mittels eines Regenerationsmittels, das in einer Richtung durch den Ionenaustauscher fließt, die der Richtung entgegengesetzt ist, in der die dem Ionenaustausch zu unterziehende Lösung beim Gebrauch des Ionenaustauschers hindurchfließt. In den Zeichnungen der Fig. 1b ist die Richtung, in der das Regenerationsmittel durch den Ionenaustauscher fließt, die Richtung von unten nach oben. Die Regeneration ist in Fig. 1b in der zweiten Zeichnung von links dargestellt.

Aufgabe des Regenerationsmittels ist es, die vom Ionenaustauscherharz zurückgehaltenen Ionen gegen H⁺ auszutauschen, um das Ionenaustauscherharz zu regenerieren. Dabei steigt im Regenerationsmittel die Konzentration der ursprünglich vom Ionenaustauscherharz zurückgehaltenen Ionen, während die Konzentration an H⁺ sinkt. Das heißt, dass das Vermögen des Regenerationsmittels schwächer wird, während es zum Regenerieren durch den Ionenaustauscher fließt. Entsprechend ist die Regeneration auf der Seite, auf der das Regenerationsmittel den Ionenaustauscher verlässt und als Abfall entsorgt wird, unvollständig, so dass auf dieser Seite nach Abschluss der Regeneration Natriumionen oder andere der durch die Regeneration eigentlich auszutauschenden Ionen zurückbleiben. Im Fall des Gleichstomverfahrens ist diese Seite die Seite, auf der auch das Produkt, d.h. einen dem Ionenaustausch unterzogene Lösung, den Ionenaustauscher verlässt. Die nach Abschluss der Regeneration zurückgebliebenen Ionen gelangen daher in das Produkt und verschlechtern so die Entsalzung des Produkts. Im Fall des Gegenstromverfahrens bleiben nach Abschluss der Regeneration auf der Seite, auf der das Produkt den Ionenaustauscher verlässt, keine Ionen zurück, so dass keine Verschleppung von Ionen in das Produkt erfolgt. Entsprechend kommt es zu keiner Verschlechterung der Entsalzung, wie es beim Gleichstromverfahren der Fall ist.

Weitere Verbesserungen sind durch Optimierung der Geometrie des Ionenaustauschers sowie der Austauschparameter möglich. Die so bereitgestellte Sulfobernsteinsäurelösung wird als Sulfobernsteinsäurematerial für die Herstellung des erfindungsgemäßen Absorbers oder Superabsorbers verwendet.

Alternativ oder zusätzlich zur Verwendung eines Kationenaustauschers kann eine Entsalzung mittels eines Membrantrennverfahrens durchgeführt werden. Dadurch ist eine Entsalzung mit geringem Zeit- und Ressourcenaufwand möglich.

In Ausführungsform der Erfindung wird die unter Verwendung von Sulfitsalzen des Natriums hergestellte und daher Natriumionen enthaltende wässrige Lösung von Sulfobernsteinsäure mit einer starken Säure angesäuert und die Lösung durch Entfernen des Natriumsalzes dieser Säure aus der Lösung mittels einer für dieses Salz bzw. die Ionen dieses Salzes durchlässige Membran entsalzt. Wenn das Ansäuern etwa mit Salzsäure durchgeführt wird, muss die Membran zumindest für Na⁺ und Cl⁻ durchlässig sein. Die so hergestellte Sulfobernsteinsäurelösung wird als Sulfobernsteinsäurematerial für die Herstellung des erfindungsgemäßen Absorbers oder Superabsorbers verwendet.

Die nachfolgend beschriebenen Beispiele dienen der Veranschaulichung vorliegender Erfindung und beschränken daher den Umfang vorliegender Erfindung in keiner Weise. Für den Fachmann ist es im Rahmen der gesamten Offenbarung offensichtlich, dass die nachfolgend beschriebenen Beispiele abgewandelt und modifiziert werden können.

Bei den Beispielen 1 bis 3 handelt es sich um Beispiele der Herstellung von Sulfobernsteinsäurelösung, welche frei oder im Wesentlichen frei von Natriumionen und anderen positiv geladenen Fremdionen ist, unter Verwendung von Schwefeldioxid, wobei die bereitgestellte Sulfobernsteinsäurelösung zur Verwendung als Sulfobernsteinsäurematerial für die Herstellung eines erfindungsgemäßen Absorbers oder Superabsorbers vorgesehen ist.

Bei Beispiel 4 handelt es sich um ein Beispiel der Herstellung von Alkalisulfobernsteinsäurelösung, welche nicht im Wesentlichen frei von Natriumionen ist, nach einem im Stand der Technik grundsätzlich bekannten Verfahren. Das in diesem Beispiel hergestellte Produkt dient als Ausgangsmaterial für die Beispiele 5 und 6.

Bei Beispiel 5 handelt es sich um ein Beispiel zur Herstellung von Sulfobernsteinsäurelösung, welche im Wesentlichen frei von Natriumionen und anderen positiv geladenen Fremdionen ist, durch Entsalzung mittels eines Membrantrennverfahrens, wobei die hergestellte Sulfobernsteinsäurelösung zur Verwendung als Sulfobernsteinsäurematerial für die Herstellung eines erfindungsgemäßen Absorbers oder Superabsorbers vorgesehen ist.

Bei Beispiel 6 handelt es sich um ein Beispiel zur Herstellung von Sulfobernsteinsäurelösung, welche im Wesentlichen frei von Natriumionen und anderen positiv geladenen Fremdionen ist, durch Entsalzung mittels Ionenaustauscher im Gegenstrom-Verfahren, wobei die hergestellte Sulfobernsteinsäurelösung zur Verwendung als Sulfobernsteinsäurematerial für die Herstellung eines erfindungsgemäßen Absorbers oder Superabsorbers vorgesehen ist.

Bei Beispiel 7 handelt es sich um ein Beispiel für die erfindungsgemäße Verwendung einer Sulfobernsteinsäurelösung, welche frei oder im Wesentlichen frei von Natriumionen und anderen positiv geladenen Fremdionen ist, als Sulfobernsteinsäurematerial zur Herstellung eines erfindungsgemäßen Absorbers oder Superabsorbers.

### Beispiel 1:

Ein Reaktionsgefäß wurde mit 100 ml Wasser befüllt und Schwefeldioxid bei Raumtemperatur bis zur Sättigung eingeleitet. Danach wurde das Reaktionsgefäß verschlossen und der Reaktionsansatz auf 150°C erhitzt. 10 g Maleinsäure wurden nun so zudosiert, dass die Temperatur im Reaktionsgefäß konstant blieb. Parallel wurde Schwefeldioxid derart zudosiert, dass der Druck im Reaktionsgefäß konstant blieb. Das Einleiten von Schwefeldioxid wurde solange fortgesetzt, bis die dünnschichtchromatographische Analyse kein Edukt mehr zeigte. Danach wurde der Reaktionsansatz auf Raumtemperatur abgekühlt und das Reaktionsgefäß vorsichtig entspannt. Die Reaktionsmasse wurde anschließend an der Atmosphäre zwei Stunden am Rückfluss erhitzt. Man erhielt 117 g einer klaren wässrigen Lösung von Sulfobernsteinsäure mit einem Sulfobernsteinsäuregehalt von 14,6 Gew.-% . Diese Lösung wurde daraufhin auf eine Lösung mit einem Sulfobernsteinsäuregehalt von 70 Gew.-% am Vakuum einrotiert. Daraufhin wurde mittels ICP-AAS der Natriumgehalt dieser Lösung ermittelt. Er betrug weniger als 5 ppm.

### Beispiel 2:

Ein Reaktionsgefäß wurde mit 130 ml Wasser befüllt und Schwefeldioxid bei Raumtemperatur bis zur Sättigung eingeleitet. Danach wurde das Reaktionsgefäß verschlossen und der Reaktionsansatz auf 170°C erhitzt. 10 g Maleinsäureanhydrid wurden nun so zudosiert, dass die Temperatur im Reaktionsgefäß konstant blieb. Parallel wurde Schwefeldioxid derart zudosiert, dass der Druck im Reaktionsgefäß konstant blieb. Das Einleiten von Schwefeldioxid wurde solange fortgesetzt, bis die dünnschichtchromatographische Analyse kein Edukt mehr zeigte. Danach wurde der Reaktionsansatz auf Raumtemperatur abgekühlt und das Reaktionsgefäß vorsichtig entspannt. Die Reaktionsmasse wurde anschließend an der Atmosphäre zwei Stunden am Rückfluss erhitzt. Man erhielt 147 g einer klaren wässrigen Lösung von Sulfobernsteinsäure mit einem Sulfobernsteinsäuregehalt von 11,6 Gew.-%. Nach dem Einrotieren dieser Lösung auf eine Lösung mit einem Sulfobernsteinsäuregehalt von 70 Gew.-% am Vakuum wurde der Natriumgehalt dieser Lösung mittels ICP-AAS bestimmt. Er betrug weniger als 5 ppm.

### Beispiel 3:

Ein Reaktionsgefäß wurde mit 500 ml Wasser befüllt und Schwefeldioxid bei Raumtemperatur bis zur Sättigung eingeleitet. Danach wurde das Reaktionsgefäß verschlossen und der Reaktionsansatz auf 180°C erhitzt. 50 g geschmolzenes Maleinsäureanhydrid wurden nun so zudosiert, dass die Temperatur im Reaktionsgefäß konstant blieb. Parallel wurde Schwefeldioxid derart zudosiert, dass der Druck im Reaktionsgefäß konstant blieb. Das Einleiten von Schwefeldioxid wurde solange fortgesetzt, bis die dünnschichtchromatographische Analyse kein Edukt mehr zeigte. Danach wurde der Reaktionsansatz auf Raumtemperatur abgekühlt und das Reaktionsgefäß vorsichtig entspannt. Die Reaktionsmasse wurde anschließend an der Atmosphäre zwei Stunden am Rückfluss erhitzt. Man erhielt 600 g einer klaren wässrigen Lösung von Sulfobernsteinsäure mit einem Sulfobernsteinsäuregehalt von 16,8 Gew.-%. Diese Lösung wurde auf eine Lösung mit einem Sulfobernsteinsäuregehalt von 70 Gew.-% am Vakuum einrotiert. Daraufhin wurde mittels ICP-AAS der Natriumgehalt dieser Lösung ermittelt. Er betrug weniger als 5 ppm.

### Beispiel 4:

130,3 g (1,25 Äquivalente) Natriumsulfit wurden bei Raumtemperatur in einem 1000 ml fassenden Reaktionsgefäß mit 480 ml Wasser unter Rühren bei Raumtemperatur gelöst (10 min). 101,4 g (1 Äquivalent) Maleinsäureanhydrid wurden als Feststoff in zehn gleich großen Portionen zugeben. Nach der vollständigen Zugabe wurde bei 65 °C für 4 h gerührt und daraufhin die Temperatur auf 95 °C erhöht und für 2 h weitergerührt. Nach dem Abkühlen auf Raumtemperatur erhielt man eine farblose, klare Lösung (ca. 600 ml).

Von dieser Lösung wurden 25 % (ca. 150 ml) entnommen und mit der doppelten Menge vollentsalztem Wasser (ca. 300 ml) verdünnt. Diese verdünnte Lösung (ca. 450 ml) wurde schließlich für Beispiel 6 herangezogen. Für Beispiel 5 wurde die unverdünnte Reaktionslösung verwendet.

### Beispiel 5:

Es wurden Reaktionslösungen aus Beispiel 4 mit 20 Gew.-%-iger Schwefelsäure auf pH 1 angesäuert und über eine Nanofiltrationsmembran (SR3D Koch Membrane Systems) bei einem Druck von 10 bar gepresst. Dabei permeierten Na⁺ und HSO₄⁻ durch die Membran, wohingegen die Sulfobernsteinsäure als das an beiden Carbonsäuregruppen protonierte Anion entsprechend der Formel 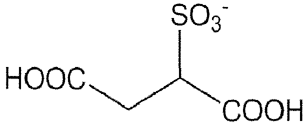 von dieser zurückgehalten wurde. Der pH-Wert der Lösung wurde dabei durch ständige Zugabe von 20 Gew.-%-iger H₂SO₄ auf einem pH-Wert von weniger als 1 gehalten. Da H₂SO₄ bei diesem Verfahren je nach Natriumgehalt in geringem Überschuss (Verhältnis Natriumgehalt : H₂SO₄-Gehalt = 1:1,2) eingesetzt werden musste, war es notwendig, diesen Überschuss an Säure nach dem Beenden der Entsalzung durch Zugabe von vollentsalztem Wasser, beispielsweise ebenfalls über die Membran abzutrennen. Danach wurde die erhaltene Lösung auf 70 Gew.-% am Vakuum einrotiert.

Der mittels ICP-AAS ermittelte Natriumgehalt der in diesem Beispiel als Produkt erhaltenen Sulfobernsteinsäurelösung mit einem Sulfobernsteinsäuregehalt von 70 Gew.-% (Gehaltsbestimmung mittels Titration) betrug 155 ppm.

### Beispiel 6:

400 ml stark saurer Kationenaustauscher (Dowex 50WX8, H+, 1,7 meq/ml) wurde in eine Säule gefüllt (3 cm Innendurchmesser, ca. 60 cm Höhe; Totvolumen ca. 150 ml). Zusätzlich wurden auf dieses Ionenaustauschermaterial als inertes Material Glaskügelchen in einer 4 cm hohen Schicht aufgebracht, um bei der Gegenstromregeneration das Ionenaustauscherbett nicht zu verändern. Daraufhin wurde die Säule mit H₂SO₄ im Gegenstrom (von unten nach oben) konditioniert. Dazu wurde fünfprozentige Schwefelsäure (ca. 700 ml) bei einer Elutionsgeschwindigkeit von ca. 300-500 ml/h durch die Säule gepumpt, bis das Eluat im Wesentlichen frei von Natriumionen war (< 10 ppm Na⁺ bestimmt mittels ICP-AAS). Daraufhin wurde die Säule mit vollentsalztem Wasser (ca. 1000 ml) gespült bis ein pH-Wert von über 5,5 erreicht war (Elutionsgeschwindigkeit ca. 500-800 ml/h). Die Reaktionslösung wurde auf den Ionenaustauscher aufgetragen und bei ca. 300-500 ml/h von oben nach unten eluiert. Das erhaltene Produkt wurde gesammelt, sobald der pH-Wert des Eluats auf unter 1,5 fiel und das Sammeln des erhaltenen Produkts wurde beendet, sobald der pH-Wert auf über 3 stieg. Daraufhin wurde diese Lösung auf 70 Gew.-% Sulfobernsteinsäure einrotiert und mittels ICP-AAS hinsichtlich des Natriumgehalts getestet. Der so ermittelte Natriumgehalt einer 70 Gew.-% Sulfobernsteinsäurelösung (Gehaltsbestimmung mittels Titration) betrug 41 ppm. Die Regeneration des Ionenaustauschers erfolgte danach im Gegenstrom, wie weiter oben bereits beschrieben.

### Beispiel 7:

Polyvinylalkohol (PVA) wurde in deionisiertem Wasser bei 90 °C unter Rühren gelöst, wobei für mindestens 6 Stunden gerührt wurde, um eine Lösung mit mindestens 10 Gew.-% PVA zu erhalten. Diese Lösung wurde mit verschiedenen Mengen an Sulfobernsteinsäurelösung (nachfolgend auch als SSA bezeichnet), die in einem der Beispiele 1 bis 3 erhalten worden war und die daher frei von Alkalimetallionen war, vermischt und die dabei erhaltenen Mischungen A bis F jeweils für 24 Stunden gerührt. Die Mischungen A bis F unterscheiden sich dabei hinsichtlich des Gewichtsverhältnisses der in ihnen enthaltenen Sulfobernsteinsäure zu dem in ihnen enthaltenen Polyvinylalkohol (Gewichtsverhältnis SSA:PVA). Das Gewichtsverhältnis SSA:PVA ist für die Mischungen A bis F in Tabelle 1 angegeben.

**Tabelle 1:**

| **Mischung** | **Gewichtsverhältnis SSA:PVA** |
|---|---|
| A | 0,1:1 |
| B | 0,2:1 |
| C | 0,3:1 |
| D | 0,4:1 |
| E | 0,5:1 |
| F | 3:1 |

Nach dem Rühren wurden diese Lösungen bei 60 °C am Vakuum einrotiert bis kein Wasser mehr abzuziehen war. Diese Masse wurde daraufhin jeweils in eine Kristallisierschale gegossen und im Trockenschrank bei je 60 °C für 3 Stunden getrocknet. Die Schichthöhe nach dem Trocknen betrug dabei etwa 5 mm. Danach erfolgte eine Temperaturbehandlung bei 120 °C für 2 Stunden, wodurch sich durch Quervernetzung ein in wässrigen Flüssigkeiten unlösliches Polymermaterial bildete. Der mittels ICP-AAS bestimmte Natriumgehalt des so hergestellten Polymermaterials betrug weniger als 5 ppm.

Das Quellverhalten des im Beispiel 7 hergestellten vernetzten Polymermaterials wurde wie folgt ermittelt:
Eine Probe mit bekannter Masse wurde in ein Filtersäckchen mit bekannter Masse (konkret in einen Teebeutel) gegeben und für 30 min in vollentsalztes Wasser eingetaucht. Danach war die Probe vollständig gequollen, d.h. sie nahm bei weiterem Versetzen mit Wasser keine größere Menge an Wasser mehr auf. Danach tropfte die Probe 30 min lang ab und wurde anschließend gewogen, um die gesamte Wasseraufnahme durch die Probe und den Teebeutel zu ermitteln. Von dieser gesamten Wasseraufnahme wurde die zuvor ermittelte Wasseraufnahme eines Teebeutels abgezogen, um die Wasseraufnahme der Probe zu berechnen.

Ein einer definierten Einwaage entsprechender kleiner Teil der so behandelten Probe wurde in einer DSC-Messeinrichtung eingefroren. Dabei gefror das freie Wasser, während das gebundene Wasser nicht gefror. Durch anschließendes Ermitteln der Schmelzenergie des gefrorenen Wassers mittels DSC (Mettler-Toledo DSC 3) konnte die Menge des freien Wassers ermittelt werden. Die Menge an gebundenem Wasser wurde berechnet, indem die Differenz zwischen der dem für die DSC-Messung verwendeten Teil der Probe entsprechenden Wasseraufnahme und der mittels DSC ermittelten Menge an freiem Wasser berechnet wurde.

In Reaktionsschema 1 ist die Quervernetzung von PVA mit Sulfobernsteinsäure schematisch dargestellt.

### Reaktionsschema 1

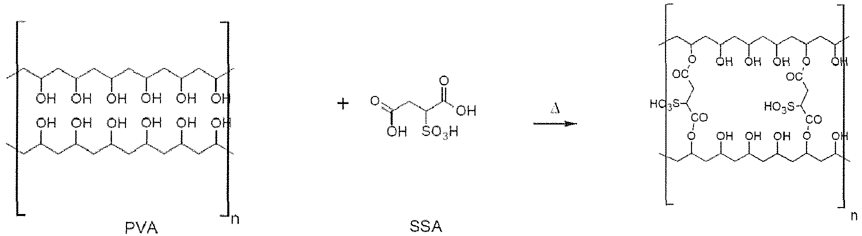

In Tabelle 2 sind für die Mischungen A bis E jeweils die Wasseraufnahme der Probe, die Menge an gebundenem Wasser sowie die Menge an freiem Wasser angegeben. Diese drei Parameter sind dabei jeweils auf die Masse des vernetzten Polymermaterials vor dem Versetzen mit Wasser, d.h. auf die Trockenmasse der Probe, bezogen.

**Tabelle 2**

| **Mischung** | **Gewichtsverhältnis SSA:PVA** | **Wasseraufnahme [Gew.-%]** | **Gebundenes Wasser [Gew.-%]** | **Freies Wasser [Gew.-%]** |
|---|---|---|---|---|
| A | 0,1:1 | 193% | 155,4% | 37,6% |
| B | 0,2:1 | 147% | 118,4% | 28,6% |
| C | 0,3:1 | 124% | 99,8% | 24,2% |
| D | 0,4:1 | 91% | 73,3% | 17,7% |
| E | 0,5:1 | 78% | 62,8% | 15,2% |
| F | 3:1 | 43% | 34,6% | 8,4% |

## Patentansprüche

1. Absorber, erhältlich durch eine Vernetzung, insbesondere eine thermische Vernetzung, eines Polymermaterials mit einem Sulfobernsteinsäurematerial,
wobei das Sulfobernsteinsäurematerial frei oder im Wesentlichen frei von Alkalimetallionen ist.

2. Absorber gemäß Anspruch 1,
wobei das Sulfobernsteinsäurematerial zusätzlich frei oder im Wesentlichen frei von Erdalkalimetallionen und/oder anderen positiv geladenen Fremdionen ist.

3. Absorber gemäß einem der vorgenannten Ansprüche,
wobei der Gehalt an Alkalimetallionen im Sulfobernsteinsäurematerial höchstens 500 ppm, bevorzugt höchstens 100 ppm, mehr bevorzugt höchstens 10 ppm ist, wobei vorzugsweise der Gehalt an Alkalimetallionen und Erdalkalimetallionen im Sulfobernsteinsäurematerial insgesamt höchstens 500 ppm, bevorzugt höchstens 100 ppm, mehr bevorzugt höchstens 10 ppm ist,
wobei insbesondere der Gehalt an positiv geladenen Fremdionen im Sulfobernsteinsäurematerial höchstens 500 ppm, bevorzugt höchstens 100 ppm, mehr bevorzugt höchstens 10 ppm ist.

4. Absorber gemäß einem der vorgenannten Ansprüche,
wobei das Sulfobernsteinsäurematerial mindestens eine der aus Sulfobernsteinsäure, Sulfobernsteinsäureanhydrid, den Sulfobernsteinsäuremonochloriden und Sulfobernsteinsäuredichlorid bestehenden Gruppe ausgewählte Verbindung umfasst, wobei das Sulfobernsteinsäurematerial bevorzugt Sulfobernsteinsäure umfasst, wobei das Sulfobernsteinsäurematerial mehr bevorzugt eine wässrige Lösung von Sulfobernsteinsäure ist,
wobei das Sulfobernsteinsäurematerial noch mehr bevorzugt eine wässrige Lösung von Sulfobernsteinsäure mit einem Sulfobernsteinsäuregehalt von 65 bis 75 Gew.-% ist,
wobei das Sulfobernsteinsäurematerial insbesondere bevorzugt eine wässrige Lösung von Sulfobernsteinsäure mit einem Sulfobernsteinsäuregehalt von 70 Gew.-% ist.

5. Absorber gemäß einem der vorgenannten Ansprüche,
wobei die Vernetzung eine Kernvernetzung und/oder eine Oberflächenvernetzung ist.

6. Absorber gemäß einem der vorgenannten Ansprüche,
wobei der Absorber biologisch abbaubar, insbesondere kompostierbar, ist, und wobei bevorzugt das Polymermaterial zumindest teilweise eine Polymerstruktur, welche Abbauschwachstellen, insbesondere Kohlenstoff-Kohlenstoff-Doppelbindungen, aufweist und welche insbesondere eine lineare Polymerstruktur ist.

7. Absorber gemäß einem der vorgenannten Ansprüche,
wobei das Polymermaterial Hydroxygruppen und/oder Aminogruppen enthält, wobei das Polymermaterial bevorzugt mindestens ein aus der aus Polyvinylalkohol, Cellulose I, Cellulose II, Chitosan, Stärke, Alginaten, Carrageenen, Dextranen, Cyclodextrinen, Copolymeren der Acrylsäure und Natriumacrylaten bestehenden Gruppe ausgewähltes Polymer enthält und
wobei das Polymermaterial weiter bevorzugt Carboxygruppen und/oder Natriumcarboxylat und/oder Kaliumcarboxylatgruppen aufweist.

8. Absorber gemäß einem der vorgenannten Ansprüche,
wobei der Absorber ein Superabsorber ist und
wobei der Absorber bei Kontakt mit einer polaren Flüssigkeit mindestens sein halbes, bevorzugt mindestens sein einfaches, mehr bevorzugt mindestens sein doppeltes Eigengewicht und/oder bis zu seinem 500-fachen, bevorzugt 1000-fachen, mehr bevorzugt 2000-fachen Eigengewicht an der polaren Flüssigkeit aufnehmen kann, wobei es sich bei der polaren Flüssigkeit bevorzugt um eine wässrige Flüssigkeit, mehr bevorzugt um eine wässrige Lösung, handelt
wobei es sich bei der polaren Flüssigkeit insbesondere um Wasser, bevorzugt um deionisiertes Wasser handelt und
wobei der Absorber bevorzugt zumindest teilweise in Form von Körnchen vorliegt, und die Körnchen mehr bevorzugt eine mittlere Korngröße (d50) von mindestens 10 µm, noch mehr bevorzugt mindestens 20 µm, insbesondere bevorzugt mindestens 50 µm und/oder mehr bevorzugt höchstens 4000 µm, noch mehr bevorzugt höchstens 2000 µm, insbesondere bevorzugt höchstens 1000 µm aufweisen.

9. Verwendung eines Absorbers gemäß einem der vorgenannten Ansprüche in einem der aus der aus Schäumen, Formkörpern, Fasern, Folien, Filmen, Kabeln, Dichtungsmaterialien, flüssigkeitsaufnehmenden Hygieneartikeln, Trägern für pflanzen- und pilzwachstumsregulierende Mittel, Wasserspeichergranulaten im Agrarsektor, gelbildenden Löschmitteln, Verpackungszusätzen, Baustoffen, Damenhygieneprodukten, Babyhygieneprodukten, Tierhygieneprodukten, Wundauflagen, Erwachseneninkontinenzprodukten, für chromatographische Trennungen verwendbaren Absorbermaterialien, insbesondere Säulenmaterialien, Trägermaterialien für Enzyme und/oder Mikroorganismen, für Membrantrennungen verwendbaren Materialien sowie im Körperpflege- und/oder Kosmetikbereich einsetzbaren Absorbermaterialien bestehenden Gruppe ausgewählten Produkt.

10. Verwendung eines Sulfobernsteinsäurematerials für die Herstellung eines Absorbers durch eine Vernetzung, insbesondere eine thermische Vernetzung, eines Polymermaterials mit dem Sulfobernsteinsäurematerial,
wobei das Sulfobernsteinsäurematerial frei oder im Wesentlichen frei von Alkalimetallionen ist.

11. Verfahren zum Herstellen eines Absorbers durch eine Vernetzung, insbesondere eine thermische Vernetzung, eines Polymermaterials mit einem Sulfobernsteinsäurematerial,
wobei das Sulfobernsteinsäurematerial frei oder im Wesentlichen frei von Alkalimetallionen ist,
wobei das Verfahren bevorzugt zumindest folgende Schritte umfasst:
(a) Bereitstellen des Polymermaterials, das optional zumindest teilweise in einem Lösungsmittel gelöst ist,
wobei es sich bei dem Lösungsmittel insbesondere um Wasser, bevorzugt um deionisiertes Wasser, handelt;
(b) Mischen des Polymermaterials mit dem Sulfobernsteinsäurematerial,
wobei das Sulfobernsteinsäurematerial frei oder im Wesentlichen frei von Alkalimetallionen ist, bevorzugt frei oder im Wesentlichen frei von Alkalimetall- und Erdalkalimetallionen, mehr bevorzugt frei oder im Wesentlichen frei von positiv geladenen Fremdionen, und
wobei das Sulfobernsteinsäurematerial optional zumindest teilweise als Lösung vorliegt,
wobei es sich bei der Lösung insbesondere um eine Lösung in Wasser, bevorzugt in deionisiertem Wasser, handelt;
(c) optional fortgesetztes mechanisches Behandeln der Mischung, insbesondere Rühren;
(d) optional Trocknen, insbesondere bei erhöhter Temperatur und/oder vermindertem Druck
(e) Durchführen einer Temperaturbehandlung zum Vernetzen des Polymermaterials mit der Sulfobernsteinsäure.

12. Verfahren gemäß Anspruch 11,
wobei die Temperaturbehandlung bevorzugt zumindest zeitweise bei einer Temperatur von mindestens 80 °C, mehr bevorzugt mindestens 100 °C, noch mehr bevorzugt mindestens 110 °C und/oder bevorzugt höchstens 160 °C, mehr bevorzugt höchstens 140 °C, noch mehr bevorzugt höchstens 130 °C, insbesondere bevorzugt bei etwa 120 °C durchgeführt wird und/oder
wobei das Gewichtsverhältnis der Sulfobernsteinsäure oder des Sulfobernsteinsäurederivats und des Polymermaterials mindestens 0,01:1, bevorzugt mindestens 0,02:1, mehr bevorzugt mindestens 0,05:1, noch mehr bevorzugt mindestens 0,1:1 und/oder höchstens 5:1, bevorzugt höchstens 3:1, mehr bevorzugt höchstens 1:1, noch mehr bevorzugt höchstens 0,5:1, insbesondere bevorzugt höchstens 0,1:1 beträgt.

13. Verfahren zum Herstellen eines Sulfobernsteinsäurematerials umfassend mindestens eine aus der aus Sulfobernsteinsäure und den Monoestern und Diestern, die Sulfobernsteinsäure mit aliphatischen Alkoholen der Formel CₙH₂ₙ₊₁OH mit n = 1, 2, 3 oder 4 bilden kann, bestehenden Gruppe ausgewählten Verbindung, bevorzugt einer Lösung der mindestens einen Verbindung, insbesondere einer wässrigen Lösung der mindestens einen Verbindung, zur Verwendung als Sulfobernsteinsäurematerial in einem Verfahren gemäß einem der Ansprüche 11 oder 12,
wobei das Verfahren zumindest folgende Schritte umfasst:
(a) Umsetzen mindestens einer aus der aus Maleinsäureanhydrid, Maleinsäure, Fumarsäure, Monoestern und Diestern der Maleinsäure sowie Monoestern und Diestern der Fumarsäure bestehenden Gruppe ausgewählten Verbindung mit mindestens einem aus der aus Alkalimetalldisulfit, Erdalkalimetalldisulfit, Alkalimetallsulfit, Erdalkalimetallsulfit, Alkalimetallhydrogensulfit und Erdalkalimetallhydrogensulfit bestehenden Gruppe ausgewählten Reaktanden;
(b) optional Verseifung mittels einer Lauge oder einem stark basischen Ionenaustauscher, falls im Schritt des Umsetzens ein Monoester und/oder Diester der Maleinsäure und/oder der Fumarsäure verwendet worden ist;
(c) Entsalzung mittels eines Ionenaustauschers im Gegenstromverfahren und/oder mittels eines Membrantrennverfahrens, insbesondere eines Nanofiltrationsverfahrens.

14. Verfahren zum Herstellen eines Sulfobernsteinsäurematerials umfassend mindestens eine aus der aus Sulfobernsteinsäure und den Monoestern und Diestern, die Sulfobernsteinsäure mit aliphatischen Alkoholen der Formel CₙH₂ₙ₊₁OH mit n = 1,2, 3 oder 4 bilden kann, bestehenden Gruppe ausgewählten Verbindung, bevorzugt einer Lösung der mindestens einen Verbindung, insbesondere einer wässrigen Lösung der mindestens einen Verbindung, zur Verwendung als Sulfobernsteinsäurematerial in einem Verfahren gemäß einem der Ansprüche 11 oder 12,
wobei mindestens eine aus der aus Maleinsäureanhydrid, Maleinsäure, Fumarsäure, Monoestern und Diestern der Maleinsäure sowie Monoestern und Diestern der Fumarsäure bestehenden Gruppe ausgewählte Verbindung in Anwesenheit von Wasser mit Schwefeldioxid zur Sulfobernsteinsäure umgesetzt wird.

15. Verfahren gemäß Anspruch 14, wobei die Umsetzung in einer wässrigen Reaktionsmischung, insbesondere in einer wässrigen Reaktionslösung durchgeführt wird, in die Schwefeldioxid unter Überdruck eingeleitet wird,
wobei das Verhältnis der Stoffmenge des eingesetzten Schwefeldioxids und der Stoffmenge der eingesetzten aus Maleinsäureanhydrid, Maleinsäure, Fumarsäure, Monoestern und Diestern der Maleinsäure sowie Monoestern und Diestern der Fumarsäure bestehenden Gruppe ausgewählten Verbindung bevorzugt höchstens 1000, mehr bevorzugt höchstens 500, noch mehr bevorzugt höchstens 100 und/oder bevorzugt mindestens 1, mehr bevorzugt mindestens 5, noch mehr bevorzugt mindestens 10 ist und/oder
wobei die Temperatur der Reaktionsmischung mindestens 0 °C, bevorzugt mindestens 10 °C, mehr bevorzugt mindestens 20 °C beträgt und/oder
wobei die Temperatur der Reaktionsmischung höchstens 250 °C, bevorzugt höchstens 200 °C, mehr bevorzugt höchstens 190 °C beträgt.
